# EUROPEAN PATENT SPECIFICATION

(11) **EP 1 611 942 B1**
(45) Date of publication and mention of the grant of the patent: **16.05.2007**
(21) Application number: 04253376.0
(22) Date of filing: 07.06.2004
(51) Int. Cl.: B01F 11/00

(54) **Stirring system for cell culture**
Rührsystem für Zellkultur
Système agitateur pour culture de cellules

(43) Date of publication of application: 04.01.2006
(73) Proprietor: The Automation Partnership (Cambridge) Limited, Royston, Hertfordshire SG8 5WY (GB)
(72) Inventor: Bargh, Adrian Neil, London N8 7LS (GB)
(74) Representative: Brunner, Michael John

(56) References cited:
- EP-A- 1 378 287
- US-A- 4 125 335
- US-B1- 6 270 249

## Description

The invention relates to a system for stirring or shaking vessels containing cells which are processed in a cell culture system.

Protein production is an essential first step in many drug discovery processes. For example, E.coli and baculovirus are used routinely in the genomics biotechnology and pharmaceutical industries for protein production because their culture is simple and relatively rapid and, additionally, sufficient quantities of purified protein can be produced for screening within a few days at a relatively economic cost.

A system to automate protein production has been developed using microorganisms and insect cells and which uses one or more incubators to enable cell growth to take place under controlled conditions. One requirement of an incubator is for aeration and agitation of the contents of vessels containing cells disposed in a suitable medium and the present invention is aimed at providing a suitable system capable of carrying out stirring and/or shaking operations (see e.g. EP 1378287 A1 and US-A-6270249).

According to a first aspect of the present invention there is provided a stirring system for a cell culture system arranged to handle a plurality of cell culture vessel assemblies each of which includes a platen on which are disposed a plurality of stirring rods, and which is transversely movable across the top of the respective culture vessel to provide for stirring of one or more samples within the culture vessel, the stirring system including:
a support configured to receive plural cell culture vessel assemblies at predefined positions around an axis;
a drive rotor disposed adjacent the support, the drive rotor being mounted for eccentric movement about the axis of the support with respect to the support and having a plurality of drive connections for selectively engaging respective platens of culture vessel assemblies supported at the predefined positions to enable simultaneous stirring of samples in the plural culture vessels whose platens are engaged with the respective drive connections; and
a drive transmission system for connecting the drive rotor to a drive motor.

Such a system enables all the attached culture vessel assemblies to be stirred by a single drive system, avoiding the need for plural drives, ie individual drives for each culture vessel assembly within an incubator.

The cell culture assemblies are preferably of the type described in our European Patent Application no. 03255896.7.

A further aspect of the invention includes a stirring system for a cell culture system arranged to handle a plurality of cell culture vessel assemblies, the stirring system including:
a support configured to receive plural cell culture vessel assemblies at a plurality of predefined positions around an axis;
a drive rotor disposed adjacent the support, the drive rotor having a corresponding plurality of sets of stirring rods for disposing in use in respective culture vessel assemblies supported at the predefined positions on the support, and being mounted for eccentric movement about the axis of the support with respect to the support to vibrate the stirring rods to enable stirring of samples in the plural culture vessels simultaneously; and
   a drive transmission system for connecting the drive rotor to a drive motor

The support may be rotatable around the axis to move the cell culture vessel assemblies between a number of positions about the axis. The drive rotor may be disposed on the support.

The drive transmission system preferably includes a plurality of eccentric bearings disposed on one or both of the rotor and the support and may also include a drive pulley mounted about the axis and a drive belt disposed around the drive pulley, and each of the bearings includes a pulley around which the drive belt is disposed to drive the rotor.

Each of the drive connections preferably includes a magnetic coupling engaging a corresponding magnetic coupling on a respective culture vessel assembly movable platen. Similarly, the culture vessel assemblies may be magnetically coupled to the support.

The culture vessel assemblies are advantageously each coupled to the support by means of a socket on the support arranged to receive a corresponding plug-in portion of the culture vessel assembly.

One or more pneumatic actuators may be disposed and actuatable to disengage culture vessel assemblies from the support.

Channels are preferably provided in the support for supplying gas or liquid to the culture vessel assemblies in use. The stirring rods are also preferably solid, but alternatively may be tubular for example for connection to the channels in the culture vessel assemblies for supplying gas or liquid into the samples in the culture vessel assemblies.

The support may be configured to receive cell culture vessels in two or more rings substantially coaxial with the axis.

The invention also includes a cell culture system including an incubator having a housing and, disposed within the housing a stirring system as defined above. A plurality of stirring systems can be disposed within the incubator housing one above another.

Example of systems according to the present invention will now be described with reference to the accompanying drawings in which:-
Figure 1 illustrates in perspective from above, a stirring system according to the invention for use in an incubator;
Figure 2 illustrates the stirring system in another perspective view from one side; and
Figure 3 is an perspective view of a culture vessel assembly for use with the system shown in figures 1 and 2.

The system shown in the drawings is for use within an incubator which includes a housing in order to enable a controlled environment to be maintained and adjusted. For simplicity the housing of the incubator is not shown in the drawings as its shape and size relative to the stirring system is not material to the invention.

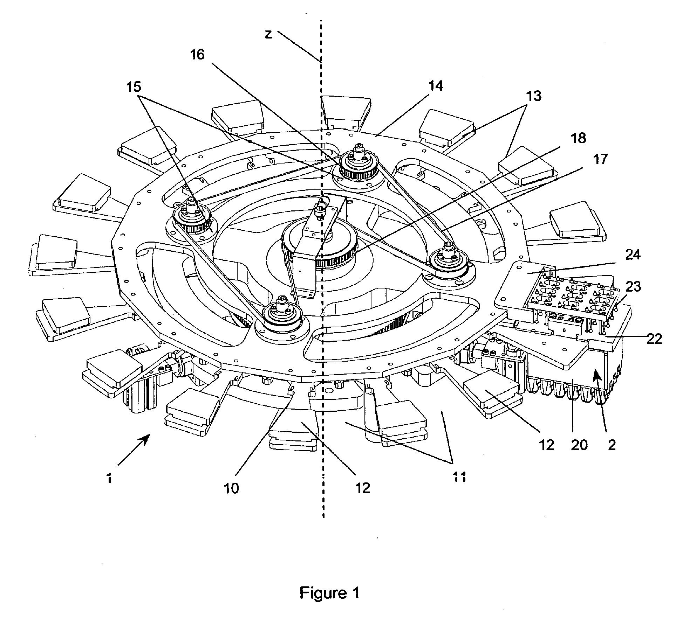

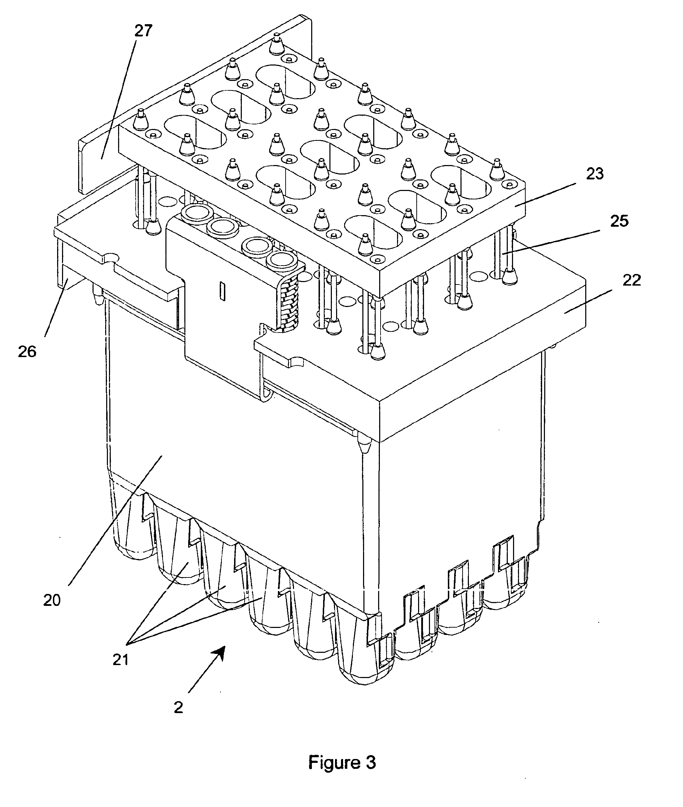

The stirring system 1 includes a rotatable support 10 in the form of a rotatable disc defining, around its periphery, a series of support locations 11 by means of protruding, generally radial, flanges 12 which define substantially parallel sides 13 for the sockets defining the support locations 11. The support is rotatable about an upright axis z. In an alternative construction (not shown) the support is fixed in position, ie non-rotatable. On the right hand side of Figure 1 there is shown a culture vessel assembly 2 of the type generally described in our European patent application no. 03255896.7, and shown in more detail in Figure 3, which has a culture vessel block 20 with multiple wells 21 for containing cells suspended or supported in a suitable medium.

The culture vessel block 20 has a detachable lid or platen 22 on which is mounted, spaced apart from the lid or platen 22, a plate 23 forming a movable platen which, as described in our European patent application no. 03255896.7, supports a plurality of stirring rods 25 extending downwardly from the platen 23, through the lid (platen) 22, and into the individual wells 21. The platen forms a plug-in connection for the culture vessel assembly on the socket 11. The construction of the culture vessel block 20 is described in more detail in our European patent application no. 03255896.7 and therefore will not be described further herein. However, it is important to note that the movable platen 23 is supported on the lid or platen 22 so as to be movable transversely relative to the lid (platen) 22 in order to cause agitation of the lower end of each stirring rod 25 within the culture vessel block wells 21.

The system 1 includes a drive rotor 14 which includes plural connectors 24, only one of which is shown (on the right hand side of the figure) which engage the platens 22,23. The connections are magnetic couplings (not shown) which engage with corresponding magnetic plate couplings 26,27 on the platens 22,23.

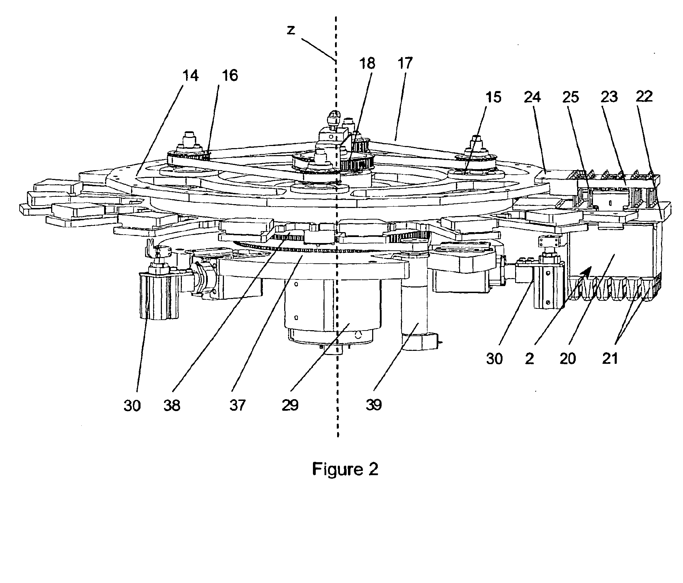

In order to move the stirring rods within the wells 21, it is necessary to move the platen 23 in an orbital fashion and this is achieved by mounting the rotor 14 for movement eccentrically with respect to the support 10. Four eccentric bearings 15 connect the rotor 14 to the support 10 (as best seen in Figure 2) and each of the bearings 15 has an associated toothed pulley 16 which is engaged by a toothed drive belt 17 which is wound around the wheels 16 and driven by a toothed pulley wheel 18 connected to a first drive motor 29 (see Fig.2). By operating the motor 29 selectively, the rotor 14 can be moved in an eccentric or orbital fashion relative to the support 10.

A second drive motor 39 (see Fig.2) is also selectively operable, via a toothed belt 37 and a toothed pulley wheel 38 connected to the support 10, to rotate the support 10, when required, so that the culture vessel assemblies 2 can be loaded into or taken out of the support locations 11 as required. Loading of the culture vessel assemblies 2 is achieved by means of a robotic system (not shown) arranged, at a suitable position around the periphery of the support 10, to present a culture vessel assembly to a selected support location 11 and pushing the culture vessel assembly 2 into the support location. Unloading is achieved by first pushing the culture vessel assembly radially to break the magnetic couplings and then removing the culture vessel assembly 2 using the robotic system. The pushing system comprises a pair of pneumatic actuators 30, disposed at different positions around the axis z and pushing directly on a culture vessel assembly 2 in use.

In an alternative construction (not shown), the couplings may be non-magnetic. The system may also allow partial de-coupling, ie the platen 23 may be de-coupled from the connector 24 (and hence the drive rotor 14) while retaining the coupling of the platen 22 on the support location 11. This allows selected culture vessel assemblies to be partially removed during stirring of the contents of the remainder of the culture vessel assemblies which remain attached to the drive rotor 14.

In a further alternative construction (not shown), the support may not be rotatable itself, and loading/unloading of culture vessel assemblies may be achieved through a robotic system capable of moving around the support.

In an alternative construction, the stirring rods and platens 23 are fixed to the rotor 14 and engageable with the culture vessel blocks 20 when they are attached to the support 10.

## Claims

1. A stirring system (1) for a cell culture system arranged to handle a plurality of cell culture vessel assemblies (2) each of which includes a platen (23) on which are disposed a plurality of stirring rods, and which is transversely movable across the top of the respective culture vessel to provide for stirring of one or more samples within the culture vessel, the stirring system including:
a support (10) configured to receive plural cell culture vessel assemblies at predefined positions around an axis (z);
a drive rotor (14) disposed adjacent the support, the drive rotor being mounted for eccentric movement about the axis (z) of the support (10) with respect to the support and having a plurality of drive connections (24) for selectively engaging respective platens (23) of culture vessel assemblies supported at the predefined positions to enable simultaneous stirring of samples in the plural culture vessels whose platens are engaged with the respective drive connections; and
a drive transmission system (15-19) for connecting the drive rotor to a drive motor (29).

2. A stirring system (1) for a cell culture system arranged to handle a plurality of cell culture vessel assemblies (2), the stirring system including:
a support (10) configured to receive plural cell culture vessel assemblies at a plurality of predefined positions around an axis (z);
a drive rotor (14) disposed adjacent the support, the drive rotor having a corresponding plurality of sets of stirring rods for disposing in use in respective culture vessel assemblies (2) supported at the predefined positions on the support (10), and being mounted for eccentric movement about the axis (z) of the support with respect to the support to vibrate the stirring rods to enable stirring of samples in the plural culture vessel assemblies (2) simultaneously; and
a drive transmission system (15-19) for connecting the drive rotor to a drive motor (29).

3. A system according to claim 1 or claim 2, wherein the support (10) is rotatable around the axis to move the cell culture vessel assemblies (2) between a number of positions about the axis (z).

4. A system according to any of claims 1 to 3, wherein the drive rotor (14) is disposed on the support (10).

5. A system according to claim 4, wherein the drive transmission system includes a plurality of eccentric bearings (15) disposed on one or both of the rotor (14) and the support (10).

6. A system according to claim 5, wherein the drive transmission system includes a drive pulley (18) mounted about the axis (z) and a drive belt (17) disposed around the drive pulley, and each of the bearings includes a pulley (16) around which the drive belt is disposed to drive the rotor (14).

7. A system according to claim 1, wherein each of the drive connections (24) includes a magnetic coupling engaging a corresponding magnetic coupling on a respective culture vessel assembly movable platen (23).

8. A system according to claim 1 or claim 2, wherein the culture vessel assemblies (2) are magnetically coupled to the support (10).

9. A system according to claim 1 or claim 2, wherein the culture vessel assemblies (2) are each coupled to the support (10) by means of a socket (11) on the support arranged to receive a corresponding plug-in portion of the culture vessel assembly.

10. A system according to any of the preceding claims, including one or more actuators disposed and actuatable to disengage culture vessel assemblies (2) from the support (10).

11. A system according to any of the preceding claims, including channels in the support for supplying gas or liquid to the culture vessel assemblies in use.

12. A system according to claim 11, wherein the stirring rods are tubular and are connected to the channels for supplying gas or liquid into the samples in the culture vessel assemblies.

13. A cell culture system including an incubator having a housing and, disposed within the housing a stirring system according to any of the preceding claims.

14. A cell culture system according to claim 13, wherein a plurality of stirring systems are disposed within the incubator housing one above another.

## Patentansprüche

1. Rührsystem (1) für ein Zellkultursystem, das angeordnet ist, um eine Mehrzahl Zellkulturgefäßanordnungen (2) zu handhaben, von denen jede eine Platte (23) umfasst, auf der eine Mehrzahl Rührstäbe angeordnet ist und die quer über die Oberseite des jeweiligen Kulturgefäßes beweglich ist, um ein Rühren einer oder mehrerer Proben in dem Kulturgefäß vorzusehen, wobei das Rührsystem umfasst:
einen Träger (10), der konfiguriert ist, um mehrere Zellkulturgefäßanordnungen an vorab definierten Positionen um eine Achse (z) aufzunehmen;
einen Antriebsrotor (14), der dem Träger benachbart angeordnet ist, wobei der Antriebsrotor zur exzentrischen Bewegung um die Achse (z) des Trägers (19) bezogen auf den Träger angebracht ist und eine Mehrzahl Antriebsverbindungen (24) aufweist, um mit jeweiligen Platten (23) von Kulturgefäßanordnungen, die an den vorab definierten Positionen getragen werden, selektiv in Eingriff zu treten, um ein gleichzeitiges Rühren von Proben in den mehreren Kulturgefäßen zu ermöglichen, deren Platten mit den jeweiligen Antriebsverbindungen in Eingriff stehen; und
ein Antriebsübertragungssystem (15-19) zum Verbinden des Antriebsrotors mit einem Antriebsmotor (29).

2. Rührsystem (1) für ein Zellkultursystem, das angeordnet ist, um eine Mehrzahl Zellkulturgefäßanordnungen (2) zu handhaben, wobei das Rührsystem umfasst:
einen Träger (10), der konfiguriert ist, um mehrere Zellkulturgefäßanordnungen an einer Mehrzahl vorab definierter Positionen um eine Achse (z) aufzunehmen;
einen Antriebsrotor (14), der dem Träger benachbart angeordnet ist, wobei der Antriebsrotor eine entsprechende Mehrzahl Sätze von Rührstäben zur Anordnung in jeweiligen Kulturgefäßanordnungen (2) bei Verwendung aufweist, die an den vorab definierten Positionen an dem Träger (10) getragen werden, und zur exzentrischen Bewegung um die Achse (z) des Trägers (19) bezogen auf den Träger angebracht ist, um die Rührstäbe vibrieren zu lassen, um ein gleichzeitiges Rühren von Proben in den mehreren Kulturgefäßen zu ermöglichen; und
ein Antriebsübertragungssystem (15-19) zum Verbinden des Antriebsrotors mit einem Antriebsmotor (29).

3. System nach Anspruch 1 oder Anspruch 2, wobei der Träger (10) um die Achse rotierbar ist, um die Zellkulturgefäßanordnungen (2) zwischen einer Anzahl von Positionen um die Achse (z) zu bewegen.

4. System nach einem der Ansprüche 1 bis 3, wobei der Antriebsrotor (14) auf dem Träger (10) angeordnet ist.

5. System nach Anspruch 4, wobei das Antriebsübertragungssystem eine Mehrzahl exzentrischer Lagerungen (15) umfasst, die auf dem Rotor (14) und/oder dem Träger (10) angeordnet sind.

6. System nach Anspruch 5, wobei das Antriebsübertragungssystem eine Antriebsscheibe (18), die auf der Achse (z) angebracht ist, und einen Antriebsriemen (17), der um die Antriebsscheibe angeordnet ist, umfasst, und wobei jede der Lagerungen eine Scheibe (16) umfasst, um die der Antriebsriemen angeordnet ist, um den Rotor (14) anzutreiben.

7. System nach Anspruch 1, wobei jede der Antriebsverbindungen (24) eine Magnetkupplung umfasst, die mit einer entsprechenden Magnetkupplung an einer jeweiligen beweglichen Kulturgefäßanordnungsplatte (23) in Eingriff tritt.

8. System nach Anspruch 1 oder 2, wobei die Kulturgefäßanordnungen (2) magnetisch mit dem Träger (10) gekoppelt sind.

9. System nach Anspruch 1 oder 2, wobei die Kulturgefäßanordnungen (2) mittels einer Fassung (11) an dem Träger, die angeordnet ist, um einen entsprechenden Einsteckabschnitt der Kulturgefäßanordnung aufzunehmen, mit dem Träger (10) gekoppelt sind.

10. System nach einem der vorhergehenden Ansprüche, umfassend einen oder mehrere Antriebe, die angeordnet und betätigbar sind, um Kulturgefäßanordnungen (2) von dem Träger (10) zu lösen.

11. System nach einem der vorhergehenden Ansprüche, umfassend Kanäle in dem Träger, um den Kulturgefäßanordnungen bei Verwendung Gase oder Flüssigkeiten zuzuführen.

12. System nach Anspruch 11, wobei die Rührstäbe rohrförmig sind und mit den Kanälen verbunden sind, um Gase oder Flüssigkeiten in die Proben in den Kulturgefäßanordnungen einzuleiten.

13. Zellkultursystem umfassend einen Inkubator mit einem Gehäuse und einem in dem Gehäuse angeordneten Rührsystem nach einem der vorhergehenden Ansprüche.

14. Zellkultursystem nach Anspruch 13, wobei eine Mehrzahl Rührsysteme übereinander in dem Inkubatorgehäuse angeordnet sind.

## Revendications

1. Système agitateur (1) pour système de culture de cellules agencé pour traiter une pluralité d'ensembles de récipients de culture de cellules (2) dont chacun comprend un plateau (23) sur lequel est disposé une pluralité de tiges agitatrices et qui est mobile transversalement en travers du sommet du récipient de culture respectif pour assurer une agitation d'un ou plusieurs échantillons dans le récipient de culture, le système agitateur comprenant :
un support (10) agencé pour recevoir plusieurs ensembles de récipients de culture de cellules à des positions définies autour d'un axe (z) ;
un rotor d'entraînement (14) disposé au voisinage du support, le rotor d'entraînement étant monté pour un mouvement excentrique autour de l'axe (z) du support (10) par rapport au support et comportant une pluralité de liaisons d'entraînement (24) pour entrer en contact sélectivement avec des plateaux respectifs (23) des ensembles de récipients de culture portés auxdites positions prédéterminées pour permettre une agitation simultanée des échantillons dans les récipients de culture dont les plateaux sont engagés avec les liaisons d'entraînement respectives ; et
un système de transmission d'entraînement (15-19) pour relier le rotor d'entraînement à un moteur d'entraînement (29).

2. Système agitateur (1) pour système de culture de cellules agencé pour traiter une pluralité d'ensembles de récipients de culture de cellules (2), le système agitateur comprenant :
un support (10) agencé pour recevoir plusieurs ensembles de récipients de culture de cellules à des positions définies autour d'un axe (z) ;
un rotor d'entraînement (14) disposé au voisinage du support, le rotor d'entraînement comprenant une pluralité d'ensembles de tiges agitatrices destinées à être disposées en utilisation dans des ensembles de récipients de culture (2) respectifs portés aux positions prédéterminées sur les supports (10) et étant montés pour un déplacement excentrique autour de l'axe (z) du support par rapport au support pour faire vibrer les tiges agitatrices pour permettre une agitation des échantillons dans les ensembles de récipients de culture (2), simultanément ; et
un système de transmission d'entraînement (15-19) pour relier le rotor d'entraînement à un moteur d'entraînement (29).

3. Système selon la revendication 1 ou 2, dans lequel le support (10) peut tourner autour de l'axe pour déplacer les ensembles de récipients de culture de cellules (2) entre plusieurs positions autour de l'axe (z).

4. Système selon l'une quelconque des revendications 1 à 3, dans lequel le rotor d'entraînement (14) est disposé sur le support (10).

5. Système selon la revendication 4, dans lequel le système de transmission d'entraînement comprend une pluralité de paliers excentriques (15) disposés sur le rotor (14), sur le support (10) ou sur les deux.

6. Système selon la revendication 5, dans lequel le système de transmission d'entraînement comprend une poulie d'entraînement (18) montée autour de l'axe (z) et une courroie d'entraînement (17) disposée autour de la poulie d'entraînement, et chacun des paliers comprend une poulie (16) autour de laquelle est disposée la courroie d'entraînement pour entraîner le rotor (14).

7. Système selon la revendication 1, dans lequel chacune des liaisons d'entraînement (24) comprend un couplage magnétique appliquant un couplage magnétique correspondant en contact sur un plateau mobile (23) d'ensemble de récipients de culture respectif.

8. Système selon la revendication 1 ou 2, dans lequel les ensembles de récipients de culture (2) sont couplés magnétiquement au support (10).

9. Système selon la revendication 1 ou 2, dans lequel chacun des ensembles de récipients de culture de cellules (2) est couplé au support (10) par une douille (11) sur le support disposée pour recevoir une partie d'engagement de l'ensemble de récipients de culture.

10. Système selon l'une quelconque des revendications précédentes, comprenant un ou plusieurs actionneurs disposés et actionnables pour désengager les ensembles de récipients de culture (2) du support (10).

11. Système selon l'une quelconque des revendications précédentes, comprenant des canaux dans le support pour fournir du gaz ou du liquide aux ensembles de récipients de culture en utilisation.

12. Système selon la revendication 11, dans lequel les tiges agitatrices sont tubulaires et sont connectées aux canaux pour fournir du gaz ou du liquide d'un échantillon dans les ensembles de récipients de culture.

13. Système de culture de cellules comprenant un incubateur comportant une enceinte et, dans l'enceinte, un système agitateur selon l'une quelconque des revendications précédentes.

14. Système de culture de cellules selon la revendication 13, dans lequel plusieurs systèmes agitateurs sont disposés dans le boîtier d'incubateur, les uns sur les autres.
